# NOUVEAU FASCICULE DE BREVET EUROPEEN

(11) **EP 0 954 332 B2**
(45) Date de publication et mention de la décision concernant l'opposition: **31.12.2014**
(45) Mention de la délivrance du brevet: 20.10.2004
(21) Numéro de dépôt: 97933747.4
(22) Date de dépôt: 15.07.1997
(51) Int. Cl.: A61K 39/295, C12N 15/45, C12N 15/35, C12N 15/50, C12N 15/38, C12N 15/47

(54) **FORMULE DE VACCIN POLYNUCLEOTIDIQUE CONTRE LES PATHOLOGIES CANINES, NOTAMMENT LES PATHOLOGIES RESPIRATOIRES ET DIGESTIVES**
IMPFSTOFFORMEL AUF POLYNUKLEOTIDBASIS ZUR BEHANDLUNG VON HUNDEKRANKHEITEN, INSBESONDERE SOLCHER DES ATMUNGS- UND VERDAUUNGSTRAKTES
POLYNUCLEOTIDE VACCINE FORMULA FOR TREATING DOG DISEASES, PARTICULARLY RESPIRATORY AND DIGESTIVE DISEASES

(30) Priorité: 19.07.1996 FR 9609401
(43) Date de publication de la demande: 10.11.1999
(73) Titulaire: MERIAL, 69002 Lyon (FR)
(72) Inventeur: AUDONNET, Jean-Christophe, F-69006 Lyon (FR); BOUCHARDON, Annabelle, F-69007 Lyon (FR); RIVIERE, Michel, F-69130 Ecully (FR)
(74) Mandataire: Cabinet Plasseraud
(86) Numéro de dépôt international: PCT/FR1997/001316
(87) Numéro de publication internationale: WO 1998/003199

(56) Documents cités:
- WO-A-95/20660
- FR-A- 2 010 678
- XIANG Z Q ET AL: "Immune response to nucleic acid vaccines to rabies virus." VIROLOGY 209 (2). 1995. 569-579, XP002029171

## Description

La présence invention est relative à une formule de vaccin permettant la vaccination des chiens contre un grand nombre de pathologies infectieuses, notamment contre le virus de la rage. Elle est également relative à une méthode de vaccination correspondante.

La pathologie infectieuse des chiens est extrêmement diversifiée et souvent difficile à contrôler en fonction des circonstances rencontrées sur le terrain.

Il existe déjà un certain nombre de vaccins, notamment contre la maladie de Carré (virus CDV), la parvovirose (virus CPV), la coronavirose (virus CCV), le complexe respiratoire ou toux des chenils (virus PI2) et la rage (rhabdovirus). Ces vaccins sont, plus généralement, des vaccins vivants constitués de souches atténuées. Cela est notamment le cas pour les vaccins de la maladie de Carré, les vaccins contre les adénoviroses canines, les vaccins contre la parvovirose et les vaccins contre le coronavirus canin.

Dans certains cas, des vaccins inactivés ont également été proposés, comme pour la rage et la coronavirose.

Ces différents vaccins sont vendus soit de façon séparée, c'est-à-dire sous forme de vaccins monovalents, soit sous forme de vaccins associés, c'est-à-dire polyvalents.

Les associations polyvalentes développées jusqu'à présent ont toujours posé des problèmes de compatibilité entre les valences et de stabilité. Il faut en effet assurer à la fois la compatibilité entre les différentes valences du vaccin, que ce soit au plan des différents antigènes utilisés et au plan des formulations elles-mêmes, notamment dans le cas où l'on combine à la fois des vaccins inactivés et des vaccins vivants. Il se pose également le problème de la conservation de tels vaccins combinés et aussi de leur innocuité notamment en présence d'adjuvant. Ces vaccins sont en général assez coûteux.

Le degré de protection, et la durée de cette protection, peuvent en outre être très variables et sont sensibles aussi aux circonstances de terrain. Cela est particulièrement vrai pour la vaccination des chiots, chez lesquels les anticorps d'origine maternelle s'opposent à l'immunisation par les vaccins inactivés et, même, par des vaccins vivants.

Il peut donc être souhaitable de perfectionner la vaccination des canidés, et notamment des chiens, tout en gardant en mémoire les contraintes économiques, qui s'opposent à l'utilisation de vaccins coûteux ou de mises en oeuvre complexes.

Des essais de vaccination contre la maladie de Carré par des préparations purifiées d'antigènes de fusion F et d'équivalents de l'hémaglutinine H en adjuvant complet de Freund ont suggéré que l'antigène F pourrait constituer un immunogène d'intérêt pour la protection contre le virus CDV (E. Norrby et al., J. of Virol. Mai 1986: 536-541), pour un vaccin de sous-unités.

Un autre article (P. de Vries et al., J. gen. Virol. 1988, 69: 2071-2083) suggère, par contre, que les protéines F et HA de CDV pourraient être intéressantes dans une vaccination selon la technique des complexes immunostimulants (ISCOMS).

Des souris immunisées par un recombinant vaccine exprimant le gène de la protéine F de CDV ont été protégés contre l'épreuve par ce virus.

Il s'agit là cependant de résultats de laboratoire, difficiles à interpréter, surtout dans des conditions de terrain.

Concernant les parvoviroses, des essais de vaccins de sous-unité contenant la protéine majeure de la capside VP2 du virus CPV obtenu par recombinaison génétique dans le baculovirus, ont permis de montrer une protection des chiens ainsi immunisés contre une épreuve par le virus CPV.

Concernant l'herpèsvirus canin CHV, des études ont été effectuées sur l'utilisation des glycoprotéines en tant que composants de vaccins de sous-unité. Ces études ont montré l'induction de réponses croisées avec d'autres herpèsvirus tels que FHV mais ne tirent pas de conclusion sur les possibilités de réaliser un vaccin protecteur.

Pour la maladie de Lyme, OspA et OspB associés induisent une protection chez la souris et le chien et OspA seul chez la souris, le hamster et le chien.

Les demandes de brevet WO-A-90 11092, WO-A-93 19183, WO-A-94 21797 et WO-A-95 20660 ont fait usage de la technique récemment développée des vaccins polynucléotidiques. On sait que ces vaccins utilisent un plasmide pate à exprimer dans les cellules de l'hôte l'antigène inséré sur le plasmide. Toutes les voies d'administration ont été proposées (intrapéritonéale, intraveineuse, intramusculaire, transcutanée, intradermique, mucosale, etc.). Différents moyens de vaccination peuvent également être utilisés, tels que ADN déposé à la surface de particules d'or et projeté de façon à pénétrer dans la peau de l'animal (Tang et al., Nature 356, 152-154, 1992) et les injecteurs par jet liquide permettant de transfecter à la fois dans la peau, le muscle, les tissus graisseux et les tissus mammaires (Furth et al., Analytical Biochemistry, 205, 365-368, 1992).

Les vaccins polynucléotidiques peuvent utiliser aussi bien des ADN nus que des ADN formulés par exemple au sein de liposomes ou de lipides cationiques.

L'art antérieur ne donne par contre aucun résultat de protection chez le chien par la méthode de la vaccination polynucléotidique contre ces maladies. Beaucoup moins de choses sont encore connues sur le coronavirus canin CCV et sur les agents responsables du complexe respiratoire.

Concernant la rage, il a été démontré une protection des souris contre épreuve virulente après un traitement par vaccin polynucléotidique exprimant le gène de la protéine G sous le contrôle du promoteur précoce du virus SV40 (Xiang et al., Virology 199, 1994,: 132-140), un résultat similaire étant atteint en utilisant le promoteur IE de CMV. L'invention se rapporte à l'utilisation d'un plasmide contenant et exprimant le gène G du virus de la rage et d'un véhicule approprié, pour la fabrication d'un vaccin pour la vaccination de l'espéce canine contre la rage. L'invention se rapporte également à l'utilisation d'un plasmide contenant et exprimant l'antigene G du virus de la rage et d'un véhicule approprié, pour la fabrication d'un vaccin induisant une réponse protectrice chez les canidés pour vacciner l'espéce canine contre le virus de la rage en association avec un vaccin entier vivant ou inactivé, recombinant ou de sous-unité dirigé contre un autre pathologie canine.

Il est décrit une formule de vaccin monovalent ou multivalent permettant d'assurer une vaccination des chiens contre un certain nombre d'agents pathogènes.

Il est décrit une telle formule de vaccin associant différentes valences tout en présentant tous les critères requis de compatibilité et de stabilité des valences entre elles.

Un autre objectif de l'invention est de fournir une telle formule de vaccin permettant d'associer différentes valences dans un même véhicule.

Un autre objectif de l'invention est de fournir une telle formule de vaccin qui soit de mise en oeuvre aisée et peu coûteuse.

Il est aussi décrit une méthode de vaccination qui permette d'accroître considérablement l'efficacité du vaccin selon l'invention ou de diminuer fortement la quantité de vaccin nécessaire, et ayant une bonne innocuité.

Le présent document décrit également une formule de vaccin contre des pathogènes des canidés, comprenant au moins deux valences de vaccin comprenant chacune un plasmide intégrant de manière à l'exprimer in vivo dans les cellules du canidé, un gène d'une valence de pathogène canin, à savoir une valence du virus de la maladie de Carré CDV et une valence du parvovirus canin CPV, les plasmides comprenant, pour chaque valence, un ou plusieurs des gènes choisis parmi le groupe consistant en HA et F pour le virus de la maladie de Carré et le gène VP2 pour le parvovirus canin.

De préférence, pour la valence de la maladie de Carré, le ou les plasmides comportent les gènes HA et F, soit insérés dans un même plasmide, soit insérés dans des plasmides différents.

Est également décrit un vaccin multivalent qui peut également comprendre une valence du coronavirus canin CCV, avec un ou des plasmides comprenant un ou plusieurs des gènes choisis parmi le groupe des gènes S et M et de préférence le gène S ou les gènes S et M. Là également, les gènes peuvent être insérés dans des plasmides différents ou regroupés dans un même plasmide dans un cadre permettant leur expression. Le vaccin bi ou trivalent précité, peut également comprendre, en outre, une valence efficace pour la prévention du complexe respiratoire, à savoir une valence de PI2 comprenant un ou des plasmides qui comprennent l'un au moins des gènes HA et F. De préférence, on prévoit d'utiliser à la fois les deux gènes HA et F.

D'autres valences intéressantes dans le cas de la présente invention peuvent donc être associées aux vaccins selon l'invention, à savoir une ou plusieurs des valences choisies dans le groupe formé par l'herpèsvirose CHV, la maladie de Lyme et la rage, les plasmides comprenant, pour chaque valence, un ou plusieurs des gènes choisis dans le groupe composé par les gènes gB, gD pour le virus CHV, les gènes OspA, OspB, et p100, pour B. Burgdorferi (maladie de Lyme), et le gène G pour la rage.

De préférence, pour l'herpèsvirose, on associe soit dans deux plasmides séparés, soit dans un seul plasmide, les deux gènes gB et gD. Pour la maladie de Lyme, on préfère le gène OspA.

Est également décrit un vaccin comprenant les valences maladie de Carré et parvovirose et qui comprendra, comme autre valence, la valence coronavirose ou, moins préférentiellement, la valence complexe respiratoire, ou ces deux valences, étant entendu que toute combinaison comprenant, une, plusieurs ou l'ensemble des valences coronavirose, complexe respiratoire, herpèsvirose, maladie de Lyme et rage peut être associée aux deux valences maladie de Carré et parvovirose.

Par valence, dans la présente invention, on entend au moins un antigène assurant une protection contre le virus du pathogène considéré, la valence pouvant contenir, à titre de sous-valence, un ou plusieurs gènes naturels ou modifiés d'une ou plusieurs souches du pathogène considéré.

Par gène d'agent pathogène, on entend non seulement le gène complet, mais aussi les séquences nucléotidiques différentes, y compris fragments, conservant la capacité à induire une réponse protectrice. La notion du gène recouvre les séquences nucléotidiques équivalentes à celles décrites précisément dans les exemples, c'est-à-dire les séquences différentes mais codant pour la même protéine. Elle recouvre aussi les séquences nucléotidiques d'autres souches du pathogène considéré, assurant une protection croisée ou une protection spécifique de souche ou de groupe de souche. Elle recouvre encore les séquences nucléotidiques qui ont été modifiées pour faciliter l'expression in vivo par l'animal hôte mais codant pour la même protéine.

Les différentes valences sont contenues dans la formulation vaccinale selon l'invention en quantité thérapeutiquement efficace.

De préférence la formule de vaccin selon l'invention pourra se présenter dans un véhicule convenable pour l'administration de préférence par voie intramusculaire, sous un volume de dose compris entre 0,1 et 5 ml, de préférence entre 0,5 et 2 ml.

La dose sera généralement comprise entre 10 ng et 1 mg, de préférence 100 ng et 500 µg, et préférentiellement entre 1 µg et 250 µg par type de plasmide.

On utilisera de préférence des plamides nus simplement placés dans le véhicule de vaccination qui sera en général de l'eau physiologique (NaCl 0,9 %), eau ultrapure, tampon TE, etc. On peut bien entendu utiliser toutes les formes de vaccins polynucléotidiques décrites dans l'art antérieur.

Chaque plasmide comprend un promoteur apte à assurer l'expression du gène inséré sous sa dépendance dans les cellules hôtes. Il s'agira en général d'un promoteur eucaryote fort et en particulier d'un promoteur précoce du cytomégalovirus CMV-IE, d'origne humaine ou murine, ou encore éventuellement d'une autre origine telle que rat, cochon, cobaye.

De manière plus générale, le promoteur pourra être soit d'origine virale, soit d'origine cellulaire. Comme promoteur viral aucre que CMV-IE, on peut citer le promoteur précoce ou tardif du virus SV 40 ou le promoteur LTR du virus du Sarcome de Rous. Il peut aussi s'agir d'un promoteur du virus dont provient le gène, par exemple le promoteur propre au gène.

Comme promoteur cellulaire, on peut citer le promoteur d'un gène du cytosquelette, tel par exemple le promoteur de la desmine (Bolmont et al., Journal of Submicroscopic Cytology and Pathology, 1990, 22, 117-122 ; et ZHENLIN et al., Gene, 1989, 78, 243-254), ou encore le promoteur de l'actine.

Lorsque plusieurs gènes sont présents dans le même plasmide, ceux-ci peuvent être présentés dans la même unité de transcription ou dans deux unités différentes.

La combinaison des différentes valences du vaccin selon l'invention peut être effectuée, de préférence, par mélange de plasmides polynucléotidiques exprimant le ou les antigène(s) de chaque valence , mais on peut également prévoir de faire exprimer des antigènes de plusieurs valences par un même plasmide.

Est également décrite une méthode de vaccination des chiens comprenant l'administration d'une dose efficace d'une formule de vaccin telle que décrite plus haut. Cette méthode de vaccination comprend l'administration d'une ou de plusieurs doses de la formule de vaccin, ces doses pouvant être administrées successivement dans un court laps de temps et/ou successivement à des moments éloignés l'un de l'autre.

Les formules de vaccin selon l'invention pourront être administrées dans le cadre de cette méthode de vaccination par les différentes voies d'administration proposées dans l'art antérieur dans le cas de la vaccination polynucléotidique et au moyen des techniques d'administration connues, la voie préférée étant la voie intramusculaire.

L'efficacité de la présentation des antigènes au système immunitaire varie en fonction des tissus. En particulier, les muqueuses de l'arbre respiratoire servent de barrière à l'entrée des pathogènes et sont associées à des tissus lymphoides qui supportent une immunité locale. L'administration d'un vaccin par contact avec les muqueuses, en particulier muqueuses buccale, pharyngée et région bronchique présente un intérêt certain pour la vaccination contre les pathologies respiratoire et digestive.

En conséquence, les voies d'administration mucosales font partie d'un mode d'administration pour l'invention, utilisant notamment la nébulisation ou spray ou l'eau de boisson. Les formules de vaccin et méthodes de vaccination selon l'invention pourront être appliquées dans ce cadre.

Sont également décrites des formules de vaccin monovalent comprenant un ou plusieurs plasmides codant pour un ou plusieurs gènes de l'un des virus ci-dessus, les gènes étant ceux décrits plus haut. En dehors de leur caractère monovalent, ces formules peuvent reprendre les caractéristiques énoncées plus haut en ce qui concerne le choix des gènes, leurs combinaisons, la composition des plasmides, les volumes de dose, les doses, etc.

Les formules de vaccin monovalent peuvent être utilisées (i) pour la préparation d'une formule de vaccin polyvalent tel que décrit plus haut (ii) à titre individuel contre la pathologie propre, (iii) associées à un vaccin d'un autre type (entier vivant ou inactivé, recombinant, sous-unité) contre une autre pathologie, ou (iv) comme rappel d'un vaccin comme décrit ci-après.

La présente invention a en effet encore pour objet l'utilisation d'un ou de plusieurs plasmides selon l'invention pour la fabrication d'un vaccin canin destiné à vacciner des animaux primo-vaccinés au moyen d'un premier vaccin classique (monovalent ou multi-valent), du type de ceux de l'art antérieur, choisi notamment dans le groupe consistant en vaccin entier vivant, vaccin entier inactivé, vaccin de sous-unité, vaccin recombinant, ce premier vaccin présentant (c'est-à-dire contenant ou pouvant exprimer) le ou les antigène(s) codé(s) par le ou les plasmide(s) ou antigène(s) assurant une protection croisée.

De manière remarquable, le vaccin polynucléotidique a un effet de rappel puissant se traduisant par une amplication de la réponse immunitaire et l'instauration d'une immunité de longue durée.

De manière générale, les vaccins de primo-vaccination pourront être choisis parmi les vaccins commerciaux disponibles auprès des différents producteurs de vaccins vétérinaires.

Il est également décrit la méthode de vaccination consistant à faire une primo-vaccination telle que décrite ci-dessus et un rappel avec une formule de vaccin selon l'invention.

Dans une forme de mise en oeuvre préférée du procédé selon l'invention, on administre dans un premier temps, à l'animal, une dose efficace du vaccin de type classique, notamment inactivé, vivant, atténué ou recombinant, ou encore un vaccin de sous-unité de façon à assurer une primo-vaccination, et, après un délai de préférence de 2 à 6 semaines, on assure l'administration du vaccin polyvalent ou monovalent selon l'invention.

Il est également décrit un kit de vaccination regroupant un vaccin de primo-vaccination tel que décrit ci-dessus et une formule de vaccin selon l'invention pour le rappel. Est également décrite une formule de vaccin selon l'invention accompagnée d'une notice indiquant l'usage de cette formule comme rappel d'une primo-vaccination telle que décrite ci-avant.

Le présent document divulgue aussi la méthode de préparation des formules de vaccin, à savoir la préparation des valences et leurs mélanges, telle qu'elle ressort de cette description.

L'invention va être maintenant décrite plus en détails à l'aide de modes de réalisation de l'invention pris en référence aux dessins annexés.

### Liste des figures

- Figure N° 1 :: Plasmide pVR1012
- Figure N° 2 :: Plasmide pAB044
- Figure N° 3 :: Plasmide pAB036
- Figure N° 4 :: Plasmide pAB024
- Figure N° 5 :: Plasmide pAB021
- Figure N° 6 :: Plasmide pAB022

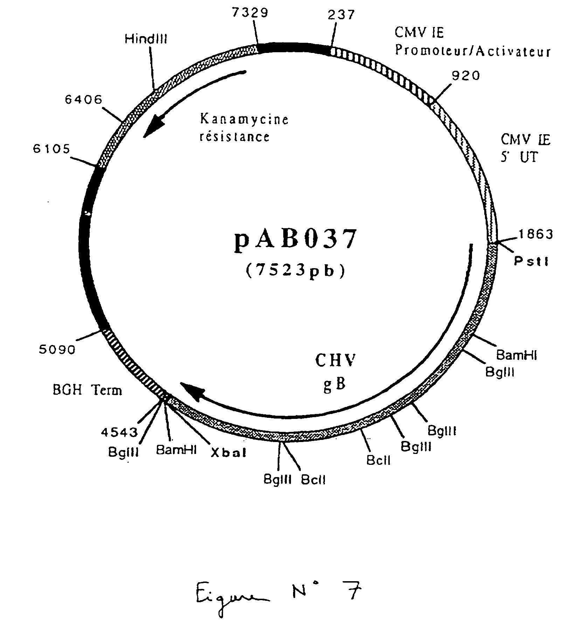

- Figure N° 7 :: Plasmide pAB037
- Figure N° 8 :: Plasmide pAB038
- Figure N° 9 :: Plasmide pAB017
- Figure N° 10 :: Plasmide pAB041

### Liste des séquences SEQ ID N°

- SEQ ID N° 1 :: Oligonucléotide AB017
- SEQ ID N° 2 :: Oligonucléotide AB018
- SEQ ID N° 3 :: Oligonucléotide AB085
- SEQ ID N° 4 :: Oligonucléotide AB086
- SEQ ID N° 5 :: Oligonucléotide AB053
- SEQ ID N° 6 :: Oligonucléotide AB054
- SEQ ID N° 7 :: Oligonucléotide AB045
- SEQ ID N° 8 :: Oligonucléotide AB048
- SEQ ID N° 9 :: Oligonucléotide AB049
- SEQ ID N° 10 :: Oligonucléotide AB050
- SEQ ID N° 11 :: Oligonucléotide AB087
- SEQ ID N° 12 :: Oligonucléotide AB088
- SEQ ID N° 13 :: Oligonucléotide AB089
- SEQ ID N° 14 :: Oligonucléotide AB090
- SEQ ID N° 15 :: Oligonucléotide AB038
- SEQ ID N° 16 :: Oligonucléotide AB039
- SEQ ID N° 17 :: Oligonucléotide AB011
- SEQ ID N° 18 :: Oligonucléotide AB012

### EXEMPLES

### Exemple 1 : Culture des virus

Les virus sont cultivés sur le système cellulaire approprié jusqu'à obtention d'un effet cytopathique. Les systèmes cellulaires à utiliser pour chaque virus sont bien connus de l'homme du métier. Brièvement, des cellules sensibles au virus utilisé, cultivées en milieu minimum essentiel de Eagle (milieu "MEM) ou un autre milieu approprié, sont inoculées avec la souche virale étudiée en utilisant une multiplicité d'infection de 1. Les cellules infectées sont alors incubées à 37°C pendant le temps nécessaire à l'apparition d'un effet cytopathique complet (en moyenne 36 heures).

### Exemple 2 : Culture des bactéries:

Les souches de *Borrelia burgdorferi sont* cultivées dans les milieux appropriés et selon les conditions bien connues de l'homme de l'art. Ces conditions et milieux sont en particulier décrits par A. Barbour (J. Biol. Med. 1984. 57. 71-75). L'extraction de l'ADN bactérien a été réalisé selon les conditions décrites par W. Simpson et al. (Infect. Immun. 1990. 58. 847-853). Les techniques usuelles décrites par J. Sambrook et al. (Molecular Cloning: A Laboratory Manual. 2nd Edition. Cold Spring Harbor Laboratory. Cold Spring Harbor. New York. 1989) peuvent également être utilisées.

### Exemple 3 : Extraction des ADNs génomiques viraux:

Après culture, le surnageant et les cellules lysées sont récoltées et la totalité de la suspension virale est centrifugée à 1000 g pendant 10 minutes à + 4°C pour éliminer les débris cellulaires. Les particules virales sont alors récoltées par ultracentrifugation à 400000 g pendant 1 heure à + 4°C. Le culot est repris dans un volume minimum de tampon (Tris 10 mM, EDTA 1 mM). Cette suspension virale concentrée est traitée par la protéinase K (100 µg/ml final) en présence de sodium dodecyl sulfate (SDS) (0,5% final) pendant 2 heures à 37°C. L'ADN viral est ensuite extrait avec un mélange de phénol/chloroforme, puis précipité avec 2 volumes d'éthanol absolu. Après une nuit à - 20°C, l'ADN est centrifugé à 10000 g pendant 15 minutes à + 4°C. Le culot d'ADN est séché, puis repris dans un volume minimum d'eau ultrapure stérile. Il peut alors être digéré par des enzymes de restriction.

### Exemple 4 : Isolement des ARNs génomiques viraux

Les virus à ARN ont été purifiés selon les techniques bien connues de l'homme du métier. L'ARN viral génomique de chaque virus a été ensuite isolé en utilisant la technique d'extraction "thiocyanate de guanidium/phénolchloroforme" décrite par P. Chomczynski et N. Sacchi (Anal. Biochem. 1987. 162. 156-159).

### Exemple 5 : Techniques de biologie moléculaire

Toutes les constructions de plasmides ont été réalisées en utilisant les techniques standards de biologie moléculaire décrites par Sambrook J. et al. (Molecular Cloning: A Laboratory Manual. 2nd Edition. Cold Spring Harbor Laboratory. Cold Spring Harbor. New York. 1989). Tous les fragments de restriction utilisés pour la présente invention ont été isolés en utilisant le kit "Geneclean" (BiO1O1 Inc. La Jolla, CA).

### Exemple 6 : Technique de RT-PCR

Des oligonucléotides spécifiques (comportant à leurs extrémités 5' des sites de restriction pour faciliter le clonage des fragments amplifiés) ont été synthétisés de telle façon qu'ils couvrent entièrement les régions codantes des gènes devant être amplifiés (voir exemples spécifiques). La réaction de transcription inverse (RT) et l'amplification en chaîne par polymérase (PCR) ont été effectuées selon les techniques standards (Sambrook J. *et al*. 1989). Chaque réaction de RT-PCR a été faite avec un couple d'amplimers spécifiques et en prenant comme matrice l'ARN génomique viral extrait. L'ADN complémentaire amplifié a été extrait au phénol/chloroforme/alcoolisoamylique (25:24:1) avant d'être digéré par les enzymes de restriction.

### Exemple 7 : plasmide pVR1012

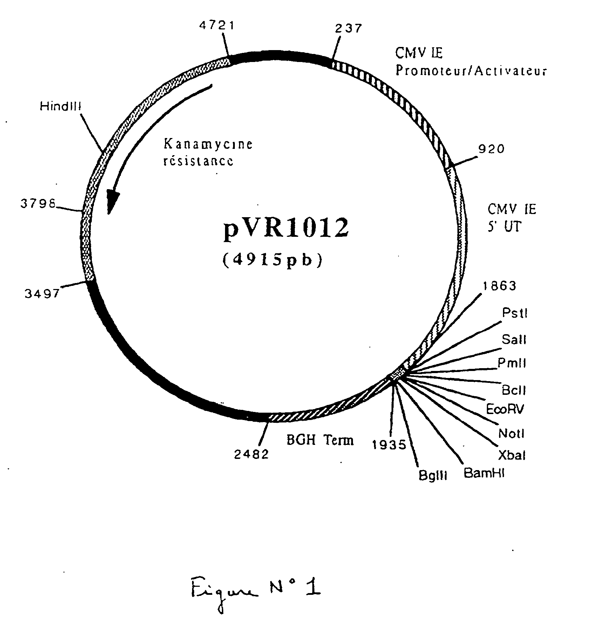

Le plasmide pVR1012 (Figure N° 1) a été obtenu auprès de Vical Inc. San Diego, CA, USA. Sa construction a été décrite dans J. Hartikka et al. (Human Gene Therapy. 1996. 7. 1205-1217).

### Exemple 8 : Construction du plasmide pAB044 (gène CDV HA)

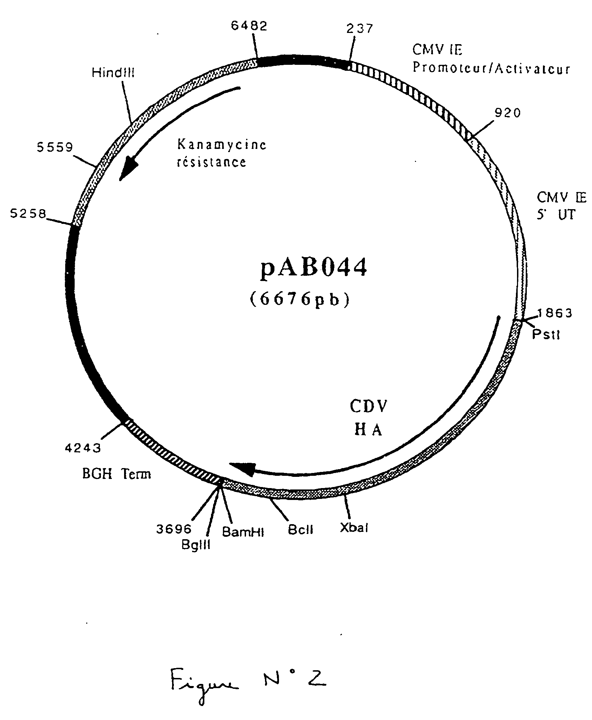

Une réaction de RT-PCR selon la technique de l'exemple 6 a été réalisée avec l'ARN génomique du virus de la maladie de Carré (CDV) (Souche Onderstepoort) (M. Sidhu et al. Virology. 1993. 193. 66-72), préparé selon la technique de l'exemple 4, et avec les oligonucléotides suivants: 
AB017 (35 mer) (SEQ ID N° 1)
5'AAAACTGCAGAATGCTCCCCTACCAAGACAAGGTG 3'
 
AB018 (37 mer) (SEQ ID N° 2)
5'CGCGGATCCTTAACGGTTACATGAGAATCTTATACGG 3'
 pour isoler le gène codant pour la glycoprotéine HA du CDV sous la forme d'un fragment Pstl-BamHl. Après purification, le produit de RT-PCR de 1835 pb a été digéré par *Pstl* et *Bam*HI pour isoler un fragment Pstl-BamHl de 1817 pb. Ce fragment a été ligaturé avec le vecteur pVR1012 (exemple 7), préalablement digéré avec *Pst*I et *Bam*HI, pour donner le plasmide pAB044 (6676 pb) (Figure N° 2).

### Exemple 9 : Construction du plasmide pAB036 (gène CDV F)

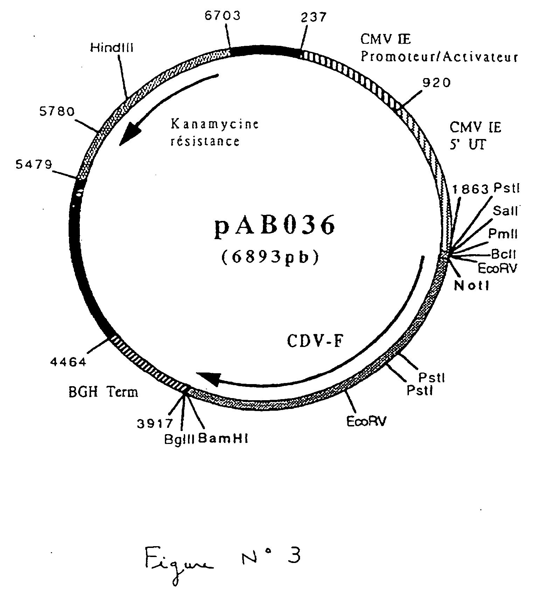

Une réaction de RT-PCR selon la technique de l'exemple 6 a été réalisée avec l'ARN génomique du virus de la maladie de Carré (CDV) (Souche Onderstepoort) (R. Driellen N° d'accès séquence sur Genbank = X65509), préparé selon la technique de l'exemple 4, et avec les oligonucléotides suivants: 
AB085 (40 mer) (SEQ ID N° 3)
5'ATAAGAAGCGGCCGCACATGCACAAGGAATCCCCAAAAG 3'
 
AB086 (32 mer) (SEQ ID N° 4)
5'CGCGGATCCACTTCAGTGTGATCTCACATAGG 3'
 pour isoler le gène codant pour la glycoprotéine F du CDV sous la forme d'un fragment Notl-BamHl. Après purification, le produit de RT-PCR de 2018 pb a été digéré par *Not*I et *Bam*HI pour isoler un fragment Notl-BamHl de 2000 pb. Ce fragment a été ligaturé avec le vecteur pVR1012 (exemple 7), préalablement digéré avec *Not*I et *Bam*HI, pour donner le plasmide pAB036 (6893 pb) (Figure N° 3).

### Exemple 10 : Construction du plasmide pAB024 (gène Parvovirus canin VP2)

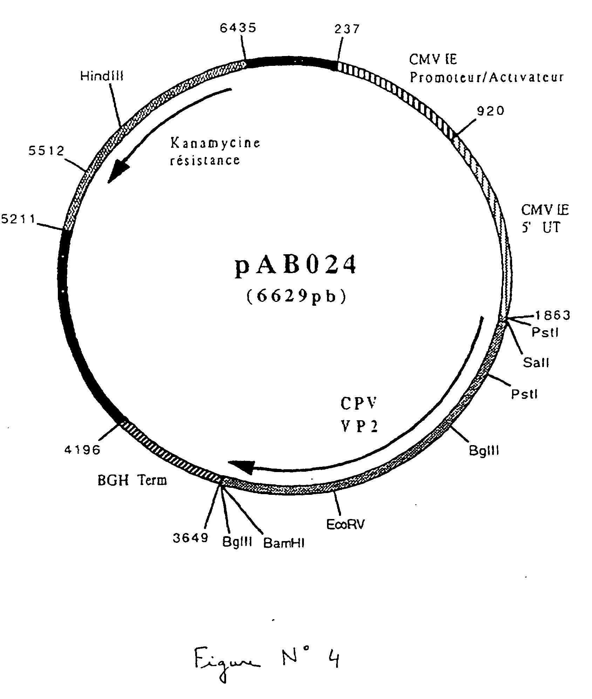

Une réaction de PCR a été réalisée avec l'ADN génomique du parvovirus canin (CPV) (Souche CPV-b) (C. Parrish N° d'accès séquence sur Genbank = M19296), préparé selon la technique de l'exemple 3, et avec les oligonucléotides suivants: 
AB053 (33 mer) (SEQ ID N° 5)
5'ACGCGTCGACATGAGTGATGGAGCAGTTCAACC 3'
 
AB054 (33 mer) (SEQ ID N° 6)
5'CGCGGATCCTTAATATAATTTTCTAGGTGCTAG 3'
 pour isoler le gène codant pour la protéine de capside VP2 (CPV VP2) sous la forme d'un fragment SalI-BamHI. Après purification, le produit de PCR de 1773 pb a été digéré par *Sal*I et *Bam*HI pour isoler un fragment Sall-BamHl de 1760 pb. Ce fragment a été ligaturé avec le vecteur pVR1012 (exemple 7), préalablement digéré avec Sall et BamHl, pour donner le plasmide pAB024 (6629 pb) (Figure N° 4).

### Exemple 11 : Construction du plasmide pAB021 (gène CCV S)

Une réaction de RT-PCR selon la technique de l'exemple 6 a été réalisée avec l'ARN génomique du coronavirus canin (CCV) (B. Horsburgh et al. J. Gen. Virol. 1992. 73. 2849-2862), préparé selon la technique de l'exemple 4, et avec les oligonucléotides suivants: 
AB045 (32 mer) (SEQ ID N° 7)
5'ACGCGTCGACATGATTGTGCTTACATTGTGCC 3'
 
AB048 (35 mer) (SEQ ID N° 8)
5'CGCGGATCCTCAGTGAACATGAACTTTTTCAATAG 3'
 pour amplifier un fragment de 4374 pb contenant le gène codant pour la glycoprotéine S du CCV sous la forme d'un fragment Sall-BamHl. Après purification, le produit de RT-PCR a été digéré par *Sal*I et *Bam*HI pour donner un fragment Sall-BamHl de 4361 pb.

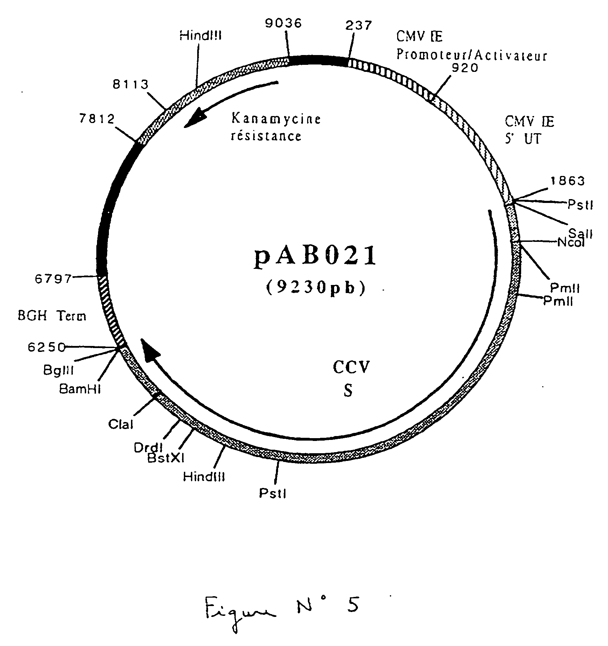

Ce fragment a été ligaturé avec le vecteur pVR1012 (exemple 7), préalablement digéré avec *Sal*I et *Bam*HI, pour donner le plasmide pAH021 (9230 pb) (Figure N° 5).

### Exemple 12 : Construction du plasmide pAB022 (gène CCV M)

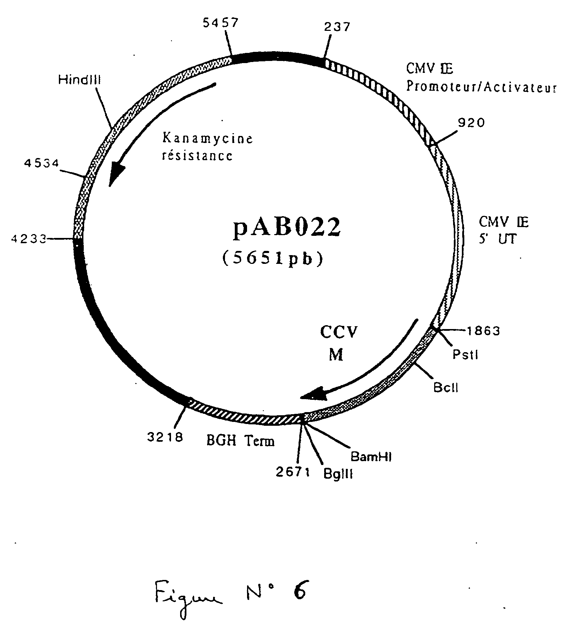

Une réaction de RT-PCR selon la technique de l'exemple 6 a été réalisée avec l'ARN génomique du coronavirus canin (CCV) (B. Horsburgh et al. J. Gen. Virol. 1992. 73. 2849-2862), préparé selon la technique de l'exemple 4, et avec les oligonucléotides suivants: 
AB049 (34 mer) (SEQ ID N° 9)
5'AAAACTGCAGAAATGAAGAAAATTTTGTTTTTAC 3'
 
AB050 (33 mer) (SEQ ID N° 10)
5'CGCGGATCCTTATACCATATGTAATAATTTTTC 3'
 pour isoler le gène codant pour la glycoprotéine M (CCV M) sous la forme d'un fragment Pstl-BamHl. Après purification, le produit de RT-PCR de 809 pb a été digéré par *Pst*I et *Bam*HI pour isoler un fragment Pstl-BamHl de 792 pb. Ce fragment a été ligaturé avec le vecteur pVR1012 (exemple 7), préalablement digéré avec *Pst*I et *Bam*HI, pour donner le plasmide pAB022 (5651 pb) (Figure N° 6).

### Exemple 13 : Construction du plasmide pAB037 (gène CHV gB)

Une réaction de PCR a été réalisée avec l'ADN génomique de l'herpèsvirus canin (CHV) (Souche Carmichael) (K. Limbach et al. J. Gen. Virol. 1994. 75. 2029-2039), préparé selon la technique de l'exemple 3, et avec les oligonucléotides suivants: 
AB087 (34 mer) (SEQ ID N° 11)
5'AAAACTGCAGAAGTATGTTTTCATTGTATCTATA 3'
 
AB088 (34 mer) (SEQ ID N° 12)
5'CTAGTCTAGATTATTAAACTTTACTTTCATTTTC 3'
 pour isoler le gène codant pour la glycoprotéine gB du virus CHV sous la forme d'un fragment Pstl-Xbal. Après purification, le produit de PCR de 2667 pb a été digéré par *Pst*I et *Xbal* pour isoler un fragment Pstl-Xbal de 2648 pb. Ce fragment a été ligaturé avec le vecteur pVR1012 (exemple 7), préalablement digéré avec *Pstl* et *Xba*I, pour donner le plasmide pAB037 (7523 pb) (Figure N ° 7).

### Exemple 14 : Construction du plasmide pAB038 (gène CHV gD)

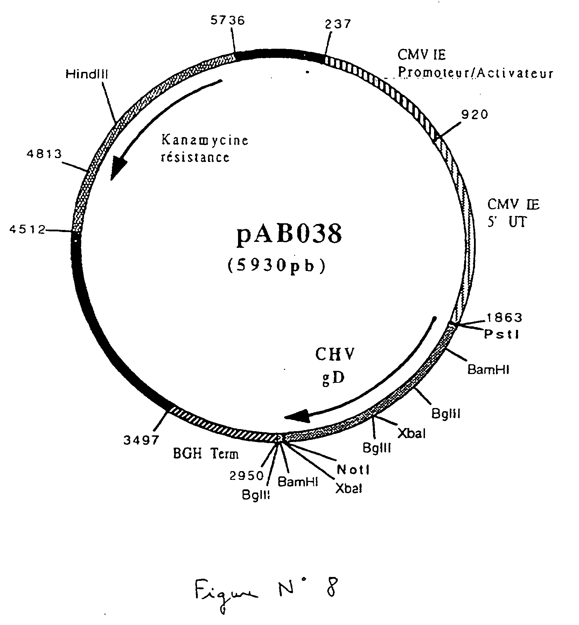

Une réaction de PCR a été réalisée avec l'ADN génomique de l'herpèsvirus canin (CHV) (Souche Carmichael) (K. Limbach et al. J. Gen. Virol. 1994. 75. 2029-2039), préparé selon la technique de l'exemple 3, et avec les oligonucléotides suivants: 
AB089 (34 mer) (SEQ ID N° 13)
5'AAAACTGCAGAAAATGATTAAACTTCTATTTATC 3'
 
AB090 (35 mer) (SEQ ID N° 14)
5'ATAAGAATGCGGCCGCAAAGGCTAAACATTTGTTG 3'
 pour isoler le gène codant pour la glycoprotéine gD du virus CHV sous la forme d'un fragment Pstl-Notl. Après purification, le produit de PCR de 1072 pb a été digéré par *Pst*I et *Not*I pour isoler un fragment Pstl-Notl de 1049 pb. Ce fragment a été ligaturé avec le vecteur pVR1012 (exemple 7), préalablement digéré avec *Pst*I et *Not*I, pour donner le plasmide pAB038 (5930 pb) (Figure N° 8).

### Exemple 15: Construction du plasmide pAB017 (gène Borrelia burgdorferi ospA)

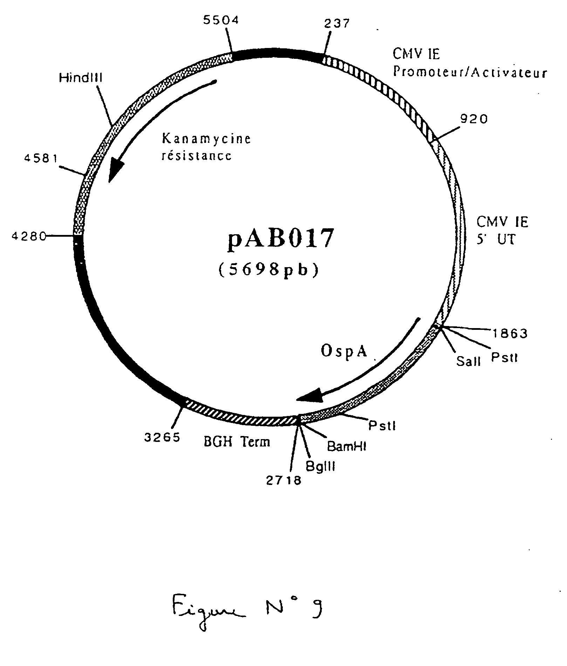

Une réaction de PCR a été réalisée avec l'ADN génomique de *Borrelia burgdorferi* (Souche B31) (S. Bergstrom et al. Mol. Microbiol. 1989. 3. 479-486.), prépaé selon la technique de l'exemple 2, et avec les oligonucléotides suivants: 
AB038 (37 mer) (SEQ ID N° 15)
5'ACGCGTCGACTATGAAAAAATATTTATTGGGAATAGG 3'
 
AB039 (34 mer) (SEQ ID N° 16)
5'CGCGGATCCCTTATTTTAAAGCGTTTTTAATTTC 3'
 pour isoler le gène codant pour la protéine de membrane OspA sous la forme d'un fragment SalI-BamHI. Après purification, le produit de PCR de 842 pb a été digéré par *Sal*I et *Bam*HI pour isoler un fragment Sall-BamHl de 829 pb. Ce fragment a été ligaturé avec le vecteur pVR1012 (exemple 7), préalablement digéré avec *Sal*I et *Bam*HI, pour donner le plasmide pAB017 (5698 pb) (Figure N° 9).

### Exemple 16 : Construction du plasmide pAB041 (gène G du virus de la rage)

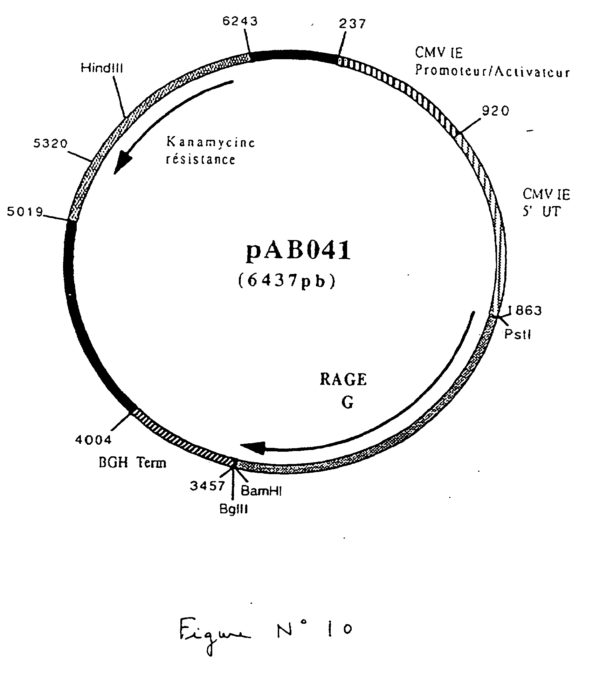

Une réaction RT-PCR selon la technique de l'exemple 6 a été réalisée avec l'ARN génomique du virus de la rage (Souche ERA) (A. Anilionis et al. Nature. 1981. 294. 275-278), préparé selon la technique de l'exemple 4, et avec tes oligonucléotides suivants: 
AB011 (33 mer) (SEQ ID N° 17)
5'AAAACTGCAGAGATGGTTCCTCAGGCTCTCCTG 3'
 
AB012 (34 mer) (SEQ ID N° 18)
5'CGCGGATCCTCACAGTCTGGTCTCACCCCCACTC 3'
 pour amplifier un fragment de 1589 pb contenant le gène codant pour la glycoprotéine G du virus de la rage. Après purification, le produit de RT-PCR a été digéré par *Pst*I et *Bam*HI pour donner un fragment Pstl-BamHl de 1578 pb. Ce fragment a été ligaturé avec le vecteur pVR1012 exemple 7), préalablement digéré avec *Pst*I et *Bam*HI, pour donner le plasmide pAB041 (6437 pb) (Figure N° 10).

### Exemple 17 : Production et purification des plasmides

Pour la préparation des plasmides destinés à la vaccination des animaux, on peut utiliser toute technique permettant d'obtenir une suspension de plasmides purifiés majoritairement sous forme superenroulée. Ces techniques sont bien connues de l'homme de l'art. On peut citer en particulier la technique de lyse alcaline suivie de deux ultracentrifugations successives sur gradient de chlorure de césium en présence de bromure d'éthidium telle que décrite dans J. Sambrook et al. (Molecular Cloning: A Laboratory Manual. 2nd Edition. Cold Spring Harbor Laboratory. Cold Spring Harbor. New York. 1989). On peut se référer également aux demandes de brevet PCT WO 95/21250 et PCT WO 96/02658 qui décrivent des méthodes pour produire à l'échelle industrielle des plasmides utilisables pour la vaccination. Pour les besoins de la fabrication des vaccins (voir exemple 17), les plasmides purifiés sont resuspendus de manière à obtenir des solutions à haute concentration ( > 2 mg/ml) compatibles avec le stockage. Pour ce faire, les plasmides sont resuspendus soit en eau ultrapure, soit en tampon TE (Tris-HCl 10 mM; EDTA 1 mM, pH 8,0).

### Exemple 18 : Fabrication des vaccins associés

Les divers plasmides nécessaires à la fabrication d'un vaccin associé sont mélangés à partir de leurs solutions concentrées (exemple 16). Les mélanges sont réalisés de telle manière que la concentration finale de chaque plasmide corresponde à la dose efficace de chaque plasmide. Les solutions utilisables pour ajuster la concentration finale du vaccin peuvent être soit une solution NACI à 0,9 % , soit du tampon PBS.

Des formulations particulières telles que les liposomes, les lipides cationiques, peuvent aussi être mises en oeuvre pour la fabrication des vaccins.

### Exemple 19 : Vaccination des chiens

Les chiens sont vaccinés avec des doses de 10 µg, 50 µg ou 250 µg par plasmide.

Les injections peuvent être réalisées à l'aiguille par voie intramusculaire. Dans ce cas, les doses vaccinales sont administrées sous des volumes de 1 ou 2 ml. Les injections peuvent être réalisées à l'aiguille par voie intradermique. Dans ce cas, les doses vaccinales sont administrées sous un volume total de 1 ml administré en 10 points de 0,1 ml ou en 20 points de 0,05 ml. Les injections intradermiques sont réalisées après avoir rasé la peau (flanc thoracique en général) ou bien au niveau d'une région anatomique relativement glabre, par exemple la face interne de la cuisse. On peut également utiliser un appareil d'injection à jet liquide pour les injections intradermiques.

## Revendications

1. Utilisation d'un plasmide contenant et exprimant le gène G du virus de la rage et d'un véhicule approprié, pour la fabrication d'un vaccin pour la vaccination de l'espèce canine contre la rage.

2. Utilisation selon la revendication 1, dans laquelle le plasmide comprend un promoteur sélectionné dans le groupe consistant en promoteur CMV-IE, promoteur précoce SV40, promoteur tarif SV40, promoteur LTR du virus du sarcome de Rous et promoteur d'un gène de cytosquelette.

3. Utilisation selon la revendication 2, dans laquelle le promoteur est un promoteur CMV-IE.

4. Utilisation selon la revendication 2, dans laquelle le promoteur d'un gène de cytosquelette est le promoteur de la desmine ou le promoteur de l'actine.

5. Utilisation d'un plasmide tel que décrit dans l'une quelconque des revendications 1 à 4, pour la fabrication d'un vaccin pour canidés pour vacciner les animaux primo-vaccinés au moyen d'un premier vaccin sélectionné dans le groupe consistant en vaccin entier vivant, vaccin entier inactivé, vaccin de sous-unité, vaccin recombinant, ce premier vaccin présentant l'antigène G.

6. Utilisation d'un plasmide contenant et exprimant l'antigène G du virus de la rage et d'un véhicule approprié, pour la fabrication d'un vaccin induisant une réponse protectrice chez les canidés pour vacciner l'espèce canine contre le virus de la rage en association avec un vaccin entier vivant ou inactivé, recombinant ou de sous-unité dirigé contre une autre pathologie canine.

7. Utilisation selon la revendication 6, dans laquelle le plasmide comprend un promoteur sélectionné dans le groupe consistant en promoteur CMV-IE, promoteur précoce SV40, promoteur tarif SV40, promoteur LTR du virus du sarcome de Rous et promoteur d'un gène de cytosquelette.

8. Utilisation selon la revendication 7, dans laquelle le promoteur est un promoteur CMV-IE.

9. Utilisation selon la revendication 7, dans laquelle le promoteur d'un gène de cytosquelette est le promoteur de la desmine ou le promoteur de l' actine.

## Patentansprüche

1. Verwendung eines Plasmids, welches das Gen G des Tollwutvirus enthält und exprimiert, und eines geeigneten Trägers für die Herstellung eines Impfstoffes, der für die Impfung der Hundegattung gegen die Tollwut bestimmt ist.

2. Verwendung nach Anspruch 1, wobei das Plasmid einen Promotor umfasst, der aus der Gruppe ausgewählt ist, die aus CMV-IE-Promotor, frühem SV40-Promotor, spätem SV40-Promotor, LTR-Promotor des Rous-Sarkom-Virus und Promotor eines Zytoskelett-Gens besteht.

3. Verwendung nach Anspruch 2, wobei der Promotor ein CMV-1E-Promotor ist.

4. Verwendung nach Anspruch 2, wobei der Promotor eines Zytoskelett-Gens der Promotor des Desmins oder der Promotor des Actins ist.

5. Verwendung eines Plasmids, das wie in einem der Ansprüche 1 bis 4 beschrieben ist, für die Herstellung eines Hundeimpfstoffes, der dazu bestimmt ist, die Tiere zu impfen, die mittels eines ersten Impfstoffes erstgeimpft wurden, der aus der Gruppe ausgewählt ist, die aus Lebend-Vollimpfstoff, inaktiviertem Vollimpfstoff, Untereinheit-Impfstoff und rekombinantem Impfstoff besteht, wobei dieser erste Impfstoff das Antigen G aufweist.

6. Verwendung eines Plasmids, welches das Antigen G des Tollwutvirus enthält und exprimiert, und eines geeigneten Trägers für die Herstellung eines Impfstoffes, der eine schützende Antwort in Hunden hervorruft, zum Impfen der Hundegattung gegen das Tollwutvirus in Verbindung mit einem Lebend-Vollimpfstoff oder inaktiviertem Vollimpfstoff, rekombinantem Impfstoff oder Untereinheit-Impfstoff, der gegen eine andere Hundekrankheit gerichtet ist.

7. Verwendung nach Anspruch 6, wobei das Plasmid einen Promotor umfasst, der aus der Gruppe ausgewählt ist, die aus CMV-1E-Promotor, frühem SV40-Promotor, spätem SV40-Promotor, LTR-Promotor des Rous-Sarkom-Virus und Promotor eines Zytoskelett-Gens besteht.

8. Verwendung nach Anspruch 7, wobei der Promotor ein CMV-IE-Promotor ist.

9. Verwendung nach Anspruch 7, wobei der Promotor eines Zytoskelett-Gens der Promotor des Desmins oder der Promotor des Actins ist.

## Claims

1. Use of a plasmid containing and expressing the G gene of the rabies virus and a suitable vehicle for the manufacture of a vaccine for vaccinating the canine species against rabies.

2. Use according to claim 1, wherein the plasmid comprises a promoter selected from the group consisting of CMV-IE promoter, SV40 early promoter, SV40 late promoter, LTR promoter of the Rous sarcoma virus and promoter of a cytoskeleton gene.

3. Use according to claim 2, wherein the promoter is a CMV-IE promoter.

4. Use according to claim 2, wherein the promoter of a cytoskeleton gene is the desmin promoter or the actin promoter.

5. Use of a plasmid as described in any one of claims 1 to 4 for the manufacture of a Canidae vaccine for vaccinating animals which have been prime vaccinated using a first vaccine selected from the group consisting of live whole vaccine, inactivated whole vaccine, subunit vaccine, recombinant vaccine, this first vaccine ·having the G antigen.

6. Use of a plasmid containing and expressing the G gene of the rabies virus and a suitable vehicle for the manufacture of a vaccine which induces a protective response in Canidae for vaccinating the canine species against the rabies virus in association with a live or Inactivated whole vaccine, recombinant vaccine or subunit vaccine directed against another canine pathology.

7. Use according to claim 6, wherein the plasmid comprises a promoter selected from the group consisting of CMV-IE promoter, SV40 early promoter, SV40 late promoter, LTR promoter of the Rous sarcoma virus and promoter of a cytoskeleton gene.

8. Use according to claim 7, wherein the promoter is a CMV-IE promoter.

9. Use according to claim 7, wherein the promoter of a cytoskeleton gene is the desmin promoter or the actin promoter.
